Europäisches Patentamt

European Patent Office

Office européen des brevets

(19)

(11) Veröffentlichungsnummer: **0 049 861**
**B1**

(12) # EUROPÄISCHE PATENTSCHRIFT

(45) Veröffentlichungstag der Patentschrift:
29.05.85

(21) Anmeldenummer: 81108018.3

(22) Anmeldetag: 06.10.81

(51) Int. Cl.⁴: **A 61 K 37/64,** C 07 K 15/14,
C 07 K 3/18

(54) **Thrombininhibitor, seine Herstellung und Verwendung.**

(30) Priorität: 09.10.80 DE 3038163

(43) Veröffentlichungstag der Anmeldung:
21.04.82 Patentblatt 82/16

(45) Bekanntmachung des Hinweises auf die Patenterteilung:
29.05.85 Patentblatt 85/22

(84) Benannte Vertragsstaaten:
AT BE CH DE FR GB IT LI LU NL SE

(56) Entgegenhaltungen:
BE - A - 877 439
DE - A - 2 243 688
DE - A - 2 725 711

CHEMICAL ABSTRACTS, Band 92, Nr. 13, 31. März 1980,
Seite 293, Nr. 106677r, Columbus, Ohio, USA
BIOLOGICAL ABSTRACTS, Band 63, Nr. 5, 1977, Nr.
24733, Columbus, Ohio, USA

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Port, Hans, Schondorferstrasse 6,
D-8120 Weilheim-Unterhausen (DE)
Erfinder: Schrenk, Jürgen, Dr., Kiefernstrasse 4,
D-8120 Weilheim (DE)
Erfinder: Wunderwald, Peter, Dr., Schulstrasse 6,
D-8121 Haunshofen (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

**Beschreibung**

Die Erfindung betrifft einen neuen Thrombininhibitor, seine Herstellung und Verwendung.

Das proteolytische Enzym Thrombin spielt im Blutgerinnungssystem eine wichtige Rolle. Bekannt ist, daß im Gerinnungssystem weiter ein spezifischer Inaktivator für das Thrombin vorkommt, welcher in Gegenwart von Heparin Thrombin zu hemmen vermag und als Antithrombin III bezeichnet wird. Die Verwendung von Heparin als Antikoagulans spielt daher in der Therapie und Forschung eine wichtige Rolle. Desgleichen ist AT III, welches auch als Heparin-Cofaktor bezeichnet wird, ein wichtiger Bestandteil von Testsystemen für die Gerinnungseigenschaften des Serums. AT III inhibiert jedoch Thrombin auch in Abwesenheit von Heparin in einer zeitabhängigen Reaktion und hemmt außer Thrombin auch Trypsin, Plasmin und Faktor Xa, ist also relativ unspezifisch.

Nunmehr wurde überraschenderweise ein neuer spezifischer Thrombininhibitor gefunden, der sich in vieler Hinsicht von AT III unterscheidet.

Der erfindungsgemäße Thrombininhibitor ist dadurch gekennzeichnet, daß er Thrombin in Gegenwart von Dextransulfat mindestens doppelt so stark wie in Gegenwart von Heparin hemmt, sich von Antithrombin III immunologisch unterscheidet, Plasmin und Trypsin praktisch nicht hemmt, ein Glykoprotein darstellt, ein Molekulargewicht von 68 000 bis 69 000 Dalton und einen isoelektrischen Punkt bei pH = 4,5 aufweist.

Da der neue Thrombininhibitor weniger stark an Heparin bindet als AT III, wird er im folgenden als AT-BM (Antithrombin Bindung Moderately to Heparin) bezeichnet.

AT-BM reagiert nicht mit AT III-Antiserum, die beiden Antithrombine zeigen also keine immunologische Kreuzreaktion. Während AT III zur optimalen Thrombinhemmung nur eine Heparinkonzentration in der Größenordnung von 0,02 USP/ml benötigt, liegt bei AT-BM die erforderliche Heparinkonzentration zur optimalen Thrombinhemmung über 1,0 USP/ml.

Während Human-AT-III unter anderem auch Faktor Xa, Plasmin und Trypsin hemmt und mit Humanthrombin unwesentlich stärker als mit Rinderthrombin reagiert, ist das neue Human-AT-BM nahezu vollständig thrombinspezifisch und inhibiert Humanthrombin deutlich stärker als Rinderthrombin. Ferner wird die Thrombinhemmung durch AT III durch Heparin stärker gefördert als durch andere Aktivatoren, wie Dextransulfat. Dagegen wird bei AT-BM mit Dextransulfat eine 2- bis 3mal so starke Thrombinhemmung erzielt als in Gegenwart von Heparin.

Während AT III Thrombin in einer zeitabhängigen Reaktion auch in vollständiger Abwesenheit von Co-Faktoren, wie Heparin hemmt, zeigt AT-BM unter diesen Umständen keinerlei Hemmwirkung.

Aufgrund der obigen Eigenschaften kann der erfindungsgemäße neue Thrombininhibitor AT-BM ähnlich wie AT III bei therapeutischen und diagnostischen Verfahren eingesetzt werden und stellt außerdem eine interessante Forschungschemikalie dar.

Die Spezifitätsunterschiede von AT-BM im Vergleich zu AT III in Gegenwart unterschiedlicher Mengen Heparin zeigt die nachstehende Tabelle 1.

Tabelle 1

Spezifitätsvergleich AT-BM/AT III

| Protease | AT III (U/ml) mit | | AT-BM (U/ml) | |
|---|---|---|---|---|
| | 0,02 | 1,75 | 0,02 | 1,75 |
| | USP Heparin/ml Testvolumen | | USP Heparin/ml Testvolumen | |
| Rinderthrombin | | | | |
| Gesamt | 22,5 | 24,4 | 6,6 | 29,0 |
| »α-Thrombin« | 25,9 | 25,6 | 3,7 | 40,3 |
| »β-Thrombin« | 20,0 | 23,5 | 0,4 | 4,5 |
| Humanthrombin | | | | |
| Gesamt | 29,4 | 30,8 | 8,4 | 74,0 |
| »α-Thrombin« | 23,0 | 31,7 | 6,2 | 101,1 |
| »β-Thrombin« | 29,0 | 37,7 | 0,1 | 18,9 |
| Faktor Xa | 5,6 | 29,5 | 0 | 0 |

Tabelle 1 (Fortsetzung)

| Protease | AT III (U/ml) mit | | AT-BM (U/ml) | |
|---|---|---|---|---|
| | 0,02 | 1,75 | 0,02 | 1,75 |
| | USP Heparin/ml Testvolumen | | USP Heparin/ml Testvolumen | |
| Plasmin | 0 | 14,5 | 0 | 0,4 |
| Trypsin | 23,5 | 16,5 | 0 | 0 |

Im normalen Humanplasma liegt das neue AT-BM neben AT III vor und liefert im Durchschnitt etwa 20% der gesamten Antithrombinaktivität, wobei die restlichen etwa 80% dem AT III zukommen. Das Verhältnis von AT-BM zu AT III kann unter bestimmten Umständen verändert sein, dies zeigen die in Tabelle 2 zusammengefaßten Versuchsergebnisse.

Tabelle 2

Antithrombin-Bestimmung mit Rinderthrombin und 1,75 USP U/ml Heparin bei 25° C

| Plasma | n | Ø Gesamt-AT U/ml | % Gesamt-AT AT-BM | AT-III |
|---|---|---|---|---|
| Normal | 5 | 8,4±0,3 | 20,8±5,4 | 79,2±5,4 |
| Schwangere | 3 | 7,5±2,5 | 14,0±2,4 | 86,0±2,4 |
| Kinder | 6 | 6,1±0,4 | 15,5±0,5 | 84,5±0,5 |
| Marcumar | 8 | 5,7±1,4 | 11,4±4,5 | 88,5±3,9 |
| Hepatitis-Verdacht | 3 | 4,6±1,9 | 10,0±5,6 | 90,0±5,6 |
| Serum | 3 | 3,9±0,7 | 32,0±4,4 | 68,0±4,4 |

Wie erwähnt ist auch die Cofaktor-Spezifität des neuen AT-BM verschieden von der von AT III. Insbesondere wird bei AT III die höchste Hemmkapazität in Gegenwart von Heparin erzielt, während bei AT-BM mit Dextransulfat noch wesentlich höhere Hemmwerte erzielbar sind. Die ermittelten Werte zeigt Tabelle 3.

Tabelle 3

Vergleich der Cofaktor-Spezifität von AT III/AT-BM bei Hemmung von Rinderthrombin

| Cofaktor (25 μg/ml) | Antithrombinaktivität U/ml von | |
|---|---|---|
| | AT III | AT-BM |
| Heparin (15 μg/ml = 1,75 USP/ml) | 27,5 | 13,2 |
| $\lambda$-Carrageenan | 5,4 | 9,3 |
| Xylan | 6,3 | 1,2 |
| Na-Dextransulfat (MG = 500 000) | 4,1 | 33,0 |
| Polystyrolsulfonat | 0 | 0 |

Auch von anderen bekannten Proteaseinhibitoren im Plasma unterscheidet sich AT-BM in seinen Eigenschaften erheblich. Dies geht aus der nachfolgenden Tabelle 4 augenscheinlich hervor.

Tabelle 4

Vergleich von AT-BM mit den bekannten Proteaseinhibitoren im menschlichen Plasma

| Inhibitor | | MG in KD[1]) | Bindung an Heparin-Seph. | Immunol. Kreuzreaktion mit AT-BM | Inhibition von | | | | |
|---|---|---|---|---|---|---|---|---|---|
| | | | | | Thrombin | Faktor Xa | Plasmin | Trypsin | Chymotrypsin |
| $\alpha$-1-Antitrypsin | | 54 | — | — | + | | + | + | + |
| $\alpha$-1-Antichymotrypsin | | 68 | — | — | | | | | + |
| Inter-$\alpha$-trypsin-Inhibitor | CRM[2] | 140 | + | — | + | | + | ± | ± |
| Säurestabiler Proteinaseinhibitor | | 34 | | | | | | | |
| $\alpha_2$-Makroglobulin | | 725 | — | — | + | | + | + | + |
| $\alpha_2$-Antiplasmin | | 70 | — | | | | + | + | |
| Antithrombin III | | 67 | + + | — | + + | + + | + | + | |
| C 1-Esterase-Inaktivator I | CRM[2] | 105 | | | | | + | — | |
| C 1-Esterase-Inaktivator II | | 96 | | | | | + | — | |
| Antithrombin-BM | | 68—69 | + | entfällt | + + | — | — | — | |

[1]) KD = Kilodalton
[2]) CRM = untereinander immunologisch kreuzreagierend

Die Gewinnung des erfindungsgemäßen Thrombininhibitors AT-BM kann aufgrund seiner unterschiedlichen Eigenschaften im Vergleich zu AT III, beispielsweise gegenüber Heparin, erfolgen, indem man die den Inhibitor enthaltende Lösung mit trägergebundenem unlöslichem Cofaktor behandelt, wobei AT III und AT-BM an den Cofaktor gebunden werden und AT-BM aufgrund der unterschiedlichen Affinität zum Cofaktor im Vergleich zu AT III von letzterem abtrennt. Als Cofaktoren können Heparin-analoge Verbindungen, wie beispielsweise Dextransulfat, verwendet werden.

Das erfindungsgemäße Verfahren zur Gewinnung des Thrombininhibitors AT-BM ist daher dadurch gekennzeichnet, daß man Serum, Plasma oder daraus gewonnene Fraktion mit unlöslichem, trägergebundenem Heparin behandelt, letzteres nach Entfernung des nichtgebundenen Materials mit einer Ionenstärke von 0,12 bis 0,36 und einem pH-Wert im Bereich von 6,5 bis 8,3 eluiert und aus dem Eluat das AT-BM gewinnt. Unter diesen Bedingungen wird AT III nicht eluiert.

Zur Einstellung des pH-Wertbereichs können die in diesem Bereich puffernden Puffersubstanzen verwendet werden. Die Konzentration des Puffers liegt zweckmäßig zwischen etwa 0,005 und 0,1 M. Die besten Ergebnisse werden bei pH-Werten zwischen 7,5 und 8,0 erhalten. Bevorzugt werden Phosphat- und Tris-Puffer.

Wie bereits erwähnt, kann das erfindungsgemäße Verfahren direkt an Serum oder Plasma durchgeführt werden. Vorzugsweise wird jedoch eine Vorreinigung nach an sich bekannten Methoden durchgeführt. Besonders geeignet erwies sich eine Plasmafraktion, welche durch Adsorption an Aluminiumhydroxidgel, Ammonsulfatfraktionierung und Dialyse erhalten wird. Eine geeignete Vorreinigungsmethode ist beispielsweise in J. Am. Chem. Soc. 68, 459—475 (1946) beschrieben. Insbesondere die dort beschriebene Fraktion IV stellt ein ohne großen Aufwand erhältliches Ausgangsmaterial dar.

Bei der Reinigung wird zweckmäßig in Gegenwart eines Sequestrierungsmittels, wie EDTA, gearbeitet. Die Konzentration desselben liegt zweckmäßig zwischen 5 und 20 mM.

Das folgende Beispiel erläutert die Erfindung weiter.

### Beispiel

1 kg Fraktion IV, erhalten gemäß J. Am. Chem. Soc. 68, 459—475 (1946), wird in 20 l 0,01 M Tris-Puffer, 0,01 M EDTA und 0,01 M NaCl, pH 8 homogenisiert und zentrifugiert.

Der opaleszente Überstand wird mit ca. 15 Vol.-% Al-Gel versetzt, 30 Minuten gerührt und abzentrifugiert.

Den Gelniederschlag wäscht man mit obigem Puffer aus und eluiert mit 1,5 Gel-Vol. 0,2 M Phosphat-Puffer, pH 7,8. Die Eluate werden mit festem Ammonsulfat auf 1,4 mol/l eingestellt, der Niederschlag wird abzentrifugiert. Der Überstand wird langsam bis 3 M Ammonsulfat gebracht und wieder zentrifugiert. Der Niederschlag wird so konzentriert wie möglich mit 0,01 Phosphat-Puffer, pH 7,8, aufgenommen und gegen die gleichen Puffer dialysiert.

Das Dialysat wird über Heparin-Sepharose® chromatographiert. Danach wird mit 2 Säulenvolumen 0,01 M Phosphat-Puffer, pH 7,8, nachgewaschen. Eluiert wird mittels des Salzgradienten von 0 bis 1,5 M NaCl in 0,01 M Phosphat-Puffer, pH 7,8. Bei 0,1 bis 0,3 M NaCl eluiert AT-BM. Das Eluat wird mit 2,8 bis 3,2 M Ammonsulfat gefällt und im kleinstmöglichen Volumen 0,01 M Tris-Puffer + 0,01 M EDTA + 0,01 M NaCl, pH 8,0 aufgenommen. Nach Dialyse gegen den gleichen Puffer wird das Präparat lyophilisiert.

**Patentansprüche**

1. Thrombininhibitor, dadurch gekennzeichnet, daß er Thrombin in Gegenwart von Dextransulfat mindestens doppelt so stark wie in Gegenwart von Heparin hemmt, sich von Antithrombin III immunologisch unterscheidet, Plasmin und Trypsin praktisch nicht hemmt, ein Glykoprotein darstellt, ein Molekulargewicht von 68 000 bis 69 000 Dalton und einen isoelektrischen Punkt bei pH = 4,5 aufweist.

2. Verfahren zur Gewinnung des Thrombininhibitors gemäß Anspruch 1, dadurch gekennzeichnet, daß man Serum, Plasma oder eine daraus gewonnene Fraktion mit unlöslichem trägergebundenem Heparin oder Dextransulfat behandelt, letzteres nach Entfernung des nichtgebundenen Materials mit einer Salzlösung mit einer Ionenstärke von 0,12 bis 0,36 und einem pH-Wert im Bereich von 6,5 bis 8,3 eluiert und aus dem Eluat den Inhibitor gewinnt.

3. Verfahren nach Anspruch 2, dadurch gekennzeichnet, daß man eine Plasmafraktion verwendet, welche durch Adsorption an Aluminiumhydroxidgel, Ammonsulfatfraktionierung und Dialyse vorgereinigt wurde.

4. Arzneimittel, dadurch gekennzeichnet, daß es Thrombininhibitor gemäß Anspruch 1 zusammen mit üblichen pharmazeutischen Zusatz- und Verdünnungsmitteln enthält.

## Claims

1. Thrombin inhibitor, characterised in that it inhibits thrombin in the presence of dextran sulphate at least twice as strongly as in the presence of heparin, differs immunologically from antithrombin III, practically does not inhibit plasmin and trypsin, is a glycoprotein, has a molecular weight of 68,000 to 69,000 Dalton and an isoelectric point of pH $= 4.5$.

2. Process for the obtaining of the thrombin inhibitor according to claim 1, characterised in that one treats serum, plasma or a fraction obtained therefrom with insoluble, carrier-bound heparin or dextran sulphate, elutes the latter, after removal of non-bound material, with a salt solution with an ion strength of 0.12 to 0.36 and a pH value in the range of 6.5 to 8.3 and obtains the inhibitor from the eluate.

3. Process according to claim 2, characterised in that one uses plasma fraction which has been prepurified by adsorption on aluminium hydroxide gel, ammonium sulphate fractionation and dialyses.

4. Medicament, characterised in that it contains thrombin inhibitor according to claim 1, together with usual pharmaceutical addition and dilution agents.

## Revendications

1. Inhibiteur de la thrombine, caractérisé en ce qu'il inhibe la thrombine en présence de sulfate de dextrane au moins deux plus fortement qu'en présence d'héparine, en ce qu'il se distingue immunologiquement de l'antithrombine III, en ce qu'il n'inhibe pratiquement pas la plasmine et la trypsine, en ce qu'il constitue une glycoprotéine, en ce qu'il présente un poids moléculaire de 68 000 à 69 000 dalton et un point isoélectrique à pH $= 4,5$.

2. Procédé d'obtention de l'inhibiteur de la thrombine suivant la revendication 1, caractérisé en ce qu'on traite le sérum, le plasma ou une fraction obtenue à partir de ceux-ci avec de l'héparine insoluble liée à un support ou du sulfate de dextrane, en ce qu'on élue cette dernière substance, après élimination de la matière non liée, avec une solution de sel ayant une force ionique de 0,12 à 0,36 et une valeur de pH dans le domaine de 6,5 à 8,3, et en ce qu'on obtient l'inhibiteur à partir de l'éluat.

3. Procédé suivant la revendication 2, caractérisé en ce qu'on utilise une fraction de plasma qui a été prépurifiée par adsorption sur un gel d'hydroxyde d'aluminium, fractionnement au sulfate d'ammonium et dialyse.

4. Médicament caractérisé en ce qu'il contient de l'inhibiteur de la thrombine suivant la revendication 1 en même temps que des additifs et diluants pharmaceutiques habituels.